# EUROPÄISCHE PATENTSCHRIFT

(11) **EP 0 524 997 B1**
(45) Veröffentlichungstag und Bekanntmachung des Hinweises auf die Patenterteilung: **10.08.1994**
(21) Anmeldenummer: 91907452.6
(22) Anmeldetag: 08.04.1991
(51) Int. Cl.: C07H 19/04, A61K 31/70, C07H 21/00, C12Q 1/68

(54) **2'-O-ALKYLNUKLEOTIDE SOWIE POLYMERE, DIE SOLCHE NUKLEOTIDE ENTHALTEN**
2'-O-ALKYL NUCLEOTIDES AND POLYMERS CONTAINING THEM
NUCLEOTIDES DE 2'-O-ALKYLE ET POLYMERES CONTENANT CES NUCLEOTIDES

(30) Priorität: 09.04.1990 DE 4011473
(43) Veröffentlichungstag der Anmeldung: 03.02.1993
(73) Patentinhaber: Europäisches Laboratorium für Molekularbiologie, D-69117 Heidelberg (DE)
(72) Erfinder: SPROAT, Brian, D-6900 Heidelberg (DE); LAMOND, Angus, D-6901 Wiesenbach (DE)
(74) Vertreter: Böhm, Brigitte, Dipl.-Chem. Dr.
(86) Internationale Anmeldenummer: EP9100665
(87) Internationale Veröffentlichungsnummer: WO9115499

(56) Entgegenhaltungen:
- EP-A- 0 260 032
- WO-A-89/12642
- Nucleic Acids Research, Band 17,Nr. 9, 11 May 1989, IRL Press Ltd., (Oxford, GB), B.S.Sproatet al.: "Highly efficient chemical synthesis of 2 '-O-methyloligoribonucleotides and tetra- biotinylated derivatives; novel probes that are resistant to degradation by RNA or DNA specific nucleases", 373-3386 siehe das ganze Dokument
- Nucleic Acids Research, Band 15, Nr.15, 11 August 1987, IRL Press Ltd., (Oxford,GB),H.Inoue et al.: "Synthesis and hybridizationstudies on two complementary nona(2'-O-methyl- ribonucleotides", Seiten 6131-6148 siehe das ganze Dokument
- Chemical Abstracts, Band 114, Nr17, 29 april 1991, (Columbus , OH. US), siehe Seite 830, Zusammenfassung 164721j, & JP, A, 02264792 (AJINOMOTO Co. INC), 29 October 1990, siehe Zusammenfassung
- A.M.IRIBARREN et al., Proc. Nath. Acad. Sci. USA 87, 7747(1990)
- G.M. LAMM et al., Nucl. Acid Research 19, 3193(1991)

## Beschreibung

Die Erfindung betrifft neue Nukleotidmonomere sowie Oligo- und Polynukleotide, die solche Monomere enthalten, ein Verfahren zu ihrer Herstellung und ihre Verwendung zur Steuerung der Genexpression, als Anti-Sense-Proben und als Arzneimittel.

Anti-Sense-Oligo- und Polynukleotide sind dem Fachmann bekannt und zusammenfassend z.B. in Spektrum der Wissenschaft (1990), Seiten 70 bis 77 beschrieben. Darunter werden die zum eigentlichen Gen komplementären Nukleotide mit gegenläufiger Sequenz verstanden. Solche Anti-Sense-Moleküle greifen regulierend in die Genexpression ein und spielen dabei eine wesentliche Rolle, ob eine in einem Gen kodierte Erbsequenz in ein Protein übersetzt wird. Die Trennung der beiden DNA-Stränge wird dabei durch eine kurze RNA-Kette, dem sogenannten Primer ausgelöst, der zuerst die DNA-Doppelhelix öffnet und der mit dem Replikationsursprung hybridisiert. Es hat sich gezeigt, daß nun die Genexpression nicht nur von der Konzentration dieser Primermoleküle abhängt, sondern von deren Verhältnis zur Anti-Sense-RNA. Auf diese Weise ist es daher möglich, in einer Zelle gezielt vorausbestimmte Gene an- und auszuschalten und so die gesamte Zellfunktion zu steuern. So ist es z.B. bereits gelungen, mittels einem Polyoma-Virus maligne transformierte Zellen dadurch wieder gesund erscheinen zu lassen, daß man in diese polyoma-transformierte Zellen Expressionsvektoren für eine Anti-Sense-RNA gegen src einschleust. Dadurch verlieren diese Zellen ihre krebsartigen Merkmale. Ähnliche Ergebnisse wurden mit dieser Anti-Sense-Technik bereits an den Onkogenen fos, ras und sys erzielt. Schließlich ist es auch bereits in Gewebekulturen gelungen, mit Anti-Sense-Oligonukleotiden die Infektion von Herpes-Viren, Influenza-Viren und dem HIV-Virus zu hemmen.
Mit Hilfe von biotinylierten Anti-Sense-Oligonukleotiden ist es auch bereits gelungen, die Ausbildung und Wirkung des Splicing-Komplexes näher zu untersuchen (S. Barabino, B. Sproat et al., The EMBO Journal 8, 4171-4178 (1989)).

Die bislang bekannten Anti-Sense-Oligo- und Polynukleotide weisen jedoch den Nachteil auf, daß diese nach dem Einschleusen in eine intakte Zelle von RNA und DNA spezifischen Nukleasen angegriffen und abgebaut werden, wodurch ihre Wirkung verlorengeht. Es ist daher bereits versucht worden, den Abbau von Poly- und Oligoribonukleotiden durch Nukleasen mittels 2′-O-Methylsubstitution zu hemmen (B. Sproat et al., Nucleic Acids Research 17 (1989), 3373-3386).

Die Erfindung hat daher zum Ziel, neue Oligo- und Polynukleotide bereitzustellen, die gegenüber einem Angriff von Nukleasen beständig sind und die mit verbesserter Spezifität an einen komplementären Nukleotidstrang binden.

Es wurde nun überraschenderweise gefunden, daß sich dieses Ziel dadurch erreichen läßt, daß man die 2′-Position mit einer Alkyloxygruppe mit mindestens 2 Kohlenstoffatomen substituiert.

Die Erfindung betrifft daher Nukleopolymere auf der Basis von 2′-O-Alkylnukleotiden der allgemeinen Formel II 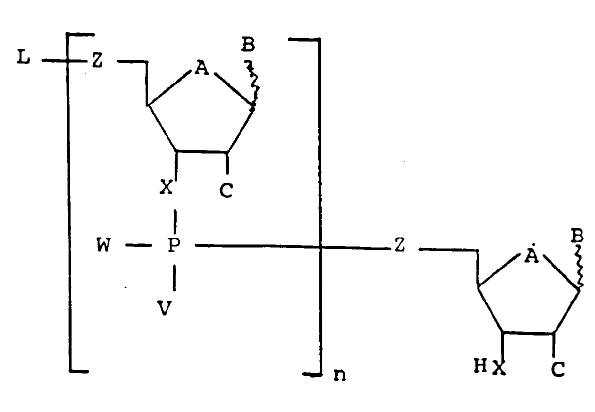
worin
B ein beliebiger, dem Fachmann bekannter Abkömmling irgendeiner Nukleosidbase und insbesondere eine Adenyl-9-yl- (A), eine Cytosin-l-yl- (C), eine Guanin-9-yl- (G), eine Uracil-1-yl- (U), eine Hypoxanthin-9-yl- (I) oder eine Thymin-1-yl-Gruppe (T) darstellt. Von den Adeninderivaten ist der 2-Aminoadenin-9-yl-Rest bevorzugt. Zweckmäßigerweise tragen ein oder mehrere der Nukleosidbasen einen Substituenten L, die die Anlagerung an bestimmte Zellteile oder Enzyme oder auch an geeignetes Chromatographiematerial erleichtert. Solche Affinitäts-Substituenten sind dem Fachmann bekannt.
A ein O-Atom oder eine CH₂-Gruppe bedeutet,
X oder Z ein O-Atom, ein S-Atom, eine NH- oder CH₂-Gruppe darstellen und sowohl gleich als auch verschieden sein können,
V und W für ein O-, S-, Se-Atom stehen, bzw. eine -OH-, -SH-, -NH₂-, Alkyl- oder Alkyloxygruppe bedeuten.
Bevorzugte Alkyl- und Alkyloxygruppen weisen 1 bis 4 Kohlenstoffatome auf und sind insbesondere -CH₃, C₂H₅ und/oder OCH₃ bzw. OC₂H₅. In einer Monomereinheit in den erfindungsgemäßen Oligo- bzw. Polynukleotiden können V und W sowohl gleich als auch verschieden sein.
L steht für ein H-Atom oder einen Partner eines Bindepaares.
C bedeutet eine Gruppe der allgemeinen Formel -O-R, worin R eine gegebenenfalls modifizierte Alkylgruppe mit insgesamt mindestens 2 C-Atomen, oder eine gegebenenfalls modifizierte Alkenyl- oder alkinylgruppe mit mindestens 2 C-Atomen bedeutet, wobei die Modifikation in einer Substitution durch einen oder mehrere Halogen-, Cyano-, Carboxy-, Hydroxy-, Nitro- oder/und MercapHalogen-, besteht. Vorzugsweise weist die Alkylgruppe 3 bis 6 und im besonderen 3 oder 4 Kohlenstoffatome auf. Beispiele für besonders geeignete Alkylgruppen sind Propyl, Butyl, jedoch sind auch modifizierte Alkylgruppen, wie Cyanomethyl, bevorzugt. Besonders bevorzugt sind Alkenylketten und im besonderen Alk-2-enylreste, wovon wiederum ein Allylrest bevorzugt ist. Beispielhaft für Alkinylgruppen kann der Propargylrest genannt werden.

Bevorzugte erfindungsgemäße Polymere weisen ein oder mehrere der zuvor geschilderten monomeren Einheiten auf, gegebenenfalls in Kombination mit anderen monomeren Einheiten, in denen -O-R gleich -O-Allyl, A gleich O, X gleich O, Z gleich O, W gleich O und V gleich OH ist und das C1-Kohlenstoffatom des Zuckers die β-Konfiguration aufweist. In einer weiteren bevorzugten Ausführungsform weisen die erfindungsgemäßen Oligo- und Polynukleotide an ihrem 3′-Ende ein 3′-Deoxyribonukleosid auf, wodurch der Angriff von 3′-Exonukleasen inhibiert und ein Abbau durch diese Enzyme zusätzlich erschwert wird.

Durch den Einbau eines Partners eines Bindepaares, z.B. aus den Paaren Antikörper/Antigen oder Biotin/Avidin bzw. Streptavidin, vorzugsweise von Biotin oder einem Dinitrophenylrest in das erfindungsgemäße Polymer ist es möglich, das erfindungsgemäße Nukleotidpolymer zu immobilisieren und eine Affinitäts-Chromatographie von Proteinen, Nukleinsäuren und/oder Protein/Nukleinsäure-Komplexen durchzuführen, die an das immobilisierte Nukleotidpolymer binden.

Die erfindungsgemäßen Oligonukleotide zeichnen sich durch eine ausgezeichnete Hybridisierung mit Nukleinsäuren aus, die eine dazu entsprechend komplementäre Zielsequenz aufweisen und sind besonders inert gegen den Abbau mittels Nukleasen, weshalb sie in lebenden Zellen eine hohe biologische Halbwertszeit aufweisen. Darüberhinaus weisen sie gegenüber dem Stand der Technik eine verringerte, nicht spezifische Bindung mit Nukleinsäure-Bindungsproteinen auf.

Die Erfindung betrifft jedoch auch Nukleotid-Monomere, die zur Synthese der erfindungsgemäßen Oligo- bzw. Polynukleotide geeignet sind und die in der 2′-Position des Zuckerteils einen gegebenenfalls durch einen oder mehrere Halogen-, Cyano-, Carboxy-, Hydroxy-, Nitro- oder/und Mercapto-Reste modifizierten Alkyloxy-, Alkenyloxy- oder Alkinyloxyrest mit mindestens 2 Kohlenstoffatomen aufweisen.

Besonders bevorzugte Reste sind O-Alk-2-enyl-Reste und im besonderen O-Allylreste. Üblicherweise weist ein solches Monomer die allgemeine Formel I 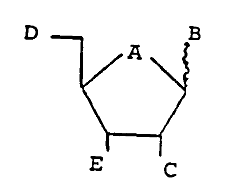
auf, worin A, B und C die zuvor definierte Bedeutung aufweisen und D und E zur Ausbildung von 3′-5′-Internukleotid-Bindungen fähige reaktive Gruppen oder die -PO₄H₂-, P₂O₇H₃-oder -P₃O₁₀H₄-Gruppe bedeuten. Solche Gruppen sind dem Fachmann bekannt und z.B. in B. Sproat et al., Nucleic Acids Research 18 (1990), 41-49 sowie zusammenfassend in E.L. Winnacker, Gene und Klone, VCH Verlagsgesellschaft mbH, Weinheim (Deutschland) (1985), insbesondere Seiten 44 bis 49 und in Froehler/Matteucci, Tetrahedron Lett. (1986), p. 469-472, beschrieben. Besonders bevorzugt als reaktive Gruppe ist die OH-Gruppe. Ebenso bevorzugt als D und/oder E sind die PO₄H₂-, -P₂O₇H₃- und die -P₃O₁₀H₄-Gruppe. Diese Mono-, Di- oder Tri-phosphate bzw. Salze der Verbindungen der allgemeinen Formel I können bevorzugt als 5′-Triphosphate, beispielsweise enzymatisch mit DNA/RNA-Polymerasen in wachsende Nucleinsäureketten eingebaut werden (vgl. z.B. Random priming, Anal.Biochem. 132 (1983) 6-13, Nick translation, J.Mol.Biol. 113 (1977) 237-251). Mit den erfindungsgemäßen reaktiven Mononukleotiden lassen sich auf an sich bekannte Weise, insbesondere an einer festen Phase die ebenfalls erfindungsgemäßen Oligo- und Polynukleotide herstellen. Die Herstellung solcher Polynukleotide aus den entsprechenden Mononukleotiden ist dem Fachmann bekannt und beispielsweise ebenfalls in den zuvor genannten Literaturstellen näher beschrieben. Die Erfindung betrifft daher auch ein Verfahren zur Herstellung von Oligo- und Polynukleotiden unter der Verwendung der erfindungsgemäßen Nukleotidmonomere.

Schließlich betrifft die Erfindung auch die Verwendung der erfindungsgemäß erhaltenen Oligo- und Polynukleotide als Anti-Sense-Proben und als Arzneimittel, insbesondere als Arzneimittel zur Behandlung von mit Viren befallenen Zellen wie z.B. dem Herpes, Influenza- oder dem AIDS-Erreger sowie zur Steuerung der Genexpression.

Die Erfindung wird durch die folgenden Beispiele näher erläutert.

### Beispiel 1

Nach dem in B. Sproat, B. Beijer und A. Iribarren in Nucleic Acids Research, Vol. 18 (1990), 41-49 beschriebenen Verfahren wurden nach der Phosphoamidit-Methode 2′-O-Allyl-oligoribonukleotide sowie 2′-O-Methyl-oligoribonukleotide mit jeweils identischer Sequenz hergestellt. Anschließend wurden, wie in A. Lamond et al. in Cell, Vol. 58 (1989), 383-390 beschriebenen Verfahren die beiden an ihrem 5′-Ende mit ³²P-Phosphat markierten Proben mit einem Kernextrakt inkubiert, der von Hela-Zellen gewonnen wurde. Beide Proben wurden dann einer Gelchromatographie unterzogen. Es zeigt sich, daß die erfindungsgemäßen 2′-O-Allyl-Oligonukleotide eine außergewöhnlich hohe spezifische und im Vergleich zu den zum Stand der Technik gehörenden 2′-O-Me-Oligonukleotide eine vernachlässigbare, nichtspezifische Bindungsaktivität.

Die Nukleotidsequenz war 5'-AIAACAIAUACUACACUUIA
Sie bindet an Human U2 RNA.

### Beispiel 2

Die gemäß Beispiel 1 hergestellten 2′-O-Allyl-Oligoribonukleotide wurden verschiedenen Nukleasen versetzt und ihrer Empfindlichkeit gegenüber enzymatischem Abbau bestimmt. Im Vergleich zu normaler, nicht modifizierter RNA mit einer identischen Sequenz zeigt sich, daß bei Verdauung mit pankreatischer RNase A, RNase CL-3, RNase T1, RNase T2 und RNase U2 die erfindungsgemäßen 2′-O-Allyl-Oligonukleotide gegenüber einem enzymatischen Angriff von Nukleasen völlig stabil sind, wohingegen natürliche RNA in allen Fällen vollständig abgebaut wird.

### Beispiel 3

3′,5′-O-(Tetraisopropyldisiloxan-1,3-diyl)-2-chlor-6-(2,6-dichlorphenoxy)purinribosid (A) wurde wie in Sproat, B.S., Beijer, B. und Iribarren, A., Nucleic Acids Research, 1990, 18, 41-49 beschrieben, synthetisiert. Die so erhaltene Verbindung A wurde dann wie nachstehend beschrieben allyliert.

### Synthese von 3′,5′-O-(Tetraisopropyldisiloxan-1,3-diyl)-2′-O-allyl-2-chlor-6-(2,6-dichlorphenoxy)purinribosid (B):

Tris(dibenzylidenaceton)dipalladium (O) (174 mg, 0,19 mmol) und 1,4-bis (Diphenylphosphin)butan (324 mg, 0,76 mmol) wur-den in trockenem Tetrahydrofuran (50 ml) suspendiert. Eine Lösung von Verbindung A (13,11 g, 19 mmol) und Allylethylcarbonat (4,95 g, 38 mmol) in 50 ml trockenem Tetrahydrofuran wurde zugesetzt und die Mischung 30 Minuten zum Rückfluß erhitzt. Silica-Gel t.l.c. in Petroläther/Ethylacetat (2:1 v/v) zeigte eine vollständige Reaktion mit einem einzigen UV-positiven Fleck von R_{f} 0,54 (Verbindung A hat R_{f} 0,41). Das Lösungsmittel wurde im Vakuum abgezogen unter Zurücklassung eines roten Sirups, der in Petroläther/Ethylacetat (9:2 v/v) gelöst und die Lösung filtriert wurde, um unlöslichen Pd-Phosphinkomplex zu entfernen. Das Produkt wurde durch präparative Flüssigkeitschromatographie auf Silica-Gel gereinigt unter Elution mit Petroläther/Ethylacetat (9:2 v/v). Die reine Verbindung B wurde so erhalten in Form eines hellgelben Schaums (12,4 g, 89,4 %). ¹³C NMR Spektrum (CDCl₃)δ: 158,12 (C6), 153,13 und 152,54 (C-2 und C-4), 144,66 (Phenyl C-1), 142,43 (C-8), 133,75 (-CH = von Allyl), 128,77 (Phenyl C-2 und C-6), 128,52 (Phenyl C-3 und C-5), 127,12 (Phenyl C-4), 120,51 (C-5), 117,0 (= CH₂ von Allyl), 88,26 (C-1′), 81,16 (C-4′), 80,6 (C-2′), 71,4 (O-CH₂- von Allyl), 69,54 (C-3′), 59,61 (C-5′), 17,19-16,63 (Isopropyl CH₃s), 13,16, 12,70 und 12,29 p.p.m. (Isopropyl CHs).

Die Verbindung B wurde weiter über verschiedene Stufen in das entsprechende Nukleotidmonomer, nämlich 5′-O-Dimethoxytrityl-N²-dimethylaminomethyliden-2′-O-allylguanosin-3′-O-(2-cyanoethyl-N,N-diisopropylphosphoramidit) unter Verwendung der in Nucleic Acids Research, 1990, 18, 41-49 beschriebenen Methode umgewandelt.

Monomere wurden in Polymere überführt wie beschrieben in Nucleic Acids Research, 1989, 17, 3373-3386.

### Beispiel 4

### Synthese von 3′,5′-O-(Tetraisopropyldisiloxan-1,3-diyl)-2′-O-propargyl-4-O-(2,6-dichlorphenyl)uridin (C):

3′,5′-0-(Tetraisopropyldisiloxan-1,3-diyl)-4-O-(2,6-dichlorphenyl)uridin (18,95 g, 30 mmol) wurden durch Verdampfen von Acetonitril im Vakuum getrocknet. Der verbleibende Schaum wurde in wasserfreiem Acetonitril (50 ml) aufgelöst, eine 80 %ige Lösung bezogen auf das Gewicht von Propargylbromid in Toluol (3,56 ml, 33 mmol) gefolgt von 2-Tert.-butylimino-2-diethylamino-1,3-dimethylperhydro-1,3,2-diazaphosphorin (9,55 ml, 33 mmol) wurden unter Rühren und Ausschluß von Feuchtigkeit zugegeben. Silicagel t.l.c. in Hexan/Ethylacetat (2:1 v/v) zeigte komplette Reaktion nach 5 Stunden mit einem UV-absorbierenden Produktpunkt von R_{f} 0,38. Das Lösungsmittel wurde im Vakuum entfernt unter Zurücklassung eines cremefarbigen Schaums. Das Produkt wurde durch präparative Flüssigchromatographie unter Verwendung von Petrolether/Dichlormethan/Ethylacetat (8:2:1, bezogen auf das Volumen) als Eluant gereinigt. Das reine Produkt C wurde erhalten als ein weißer Schaum (10,7 g, 53,3 %). ¹³C NMR-Spektrum (CDCl₃ )δ: 169,80 (C-4), 154,60 (C-2), 144,76 (Phenyl C-1), 144,41 (C-6), 128,69 (Phenyl C-2 und C-6), 128,65 (Phenyl C-3 und C-5), 127,08 (Phenyl C-4), 93,80 (C-5), 89,80 (C-1′), 81,70 (C-2′), 80,34 (C-4′), 79,54 (-C≡ von Propargyl), 74,63 (≡CH von Propargyl), 67,63 (C-3′), 59,37 (C-5′), 58,01 (OCH₂ von Propargyl), 17,36, 17,21, 16,90 und 16,73 (Isopropyl CH₃ₛ ), 13,36, 12,92, 12,84 und 12,28 p.p.m. (Isopropyl CHₛ).

Die Verbindung C konnte über verschiedene Schritte in Cytidin und Uridin-Monomere zur Festphasenpolymer-Herstellung überführt werden.

### Beispiel 5

### Synthese von 3′,5′-O-(Tetraisopropyldisiloxan-1,3-diyl)-2′-O-cyanomethyl-4-O-(2,6-dichlorphenyl)uridin (D):

Die Alkylierung wurde analog Beispiel 4 durchgeführt unter Verwendung von Bromacetonitril anstelle von Propargylbromid. Die Titelverbindung wurde erhalten als ein weißer Schaum in 55 %iger Ausbeute, R_{f} 0,51 auf Silicagel t.l.c. in Petrolether/Ethylacetat (1:1 v/v). ¹³C NMR-Spektrum (CDCl₃ )δ: 169,91 (C-4), 154,57 (C-2), 144,54 (Phenyl C-1), 143,96 (C-6), 128,69 (Phenyl C-2 und C-6), 128,60 (Phenyl C-3 und C-5), 127,12 (Phenyl C-4), 115,72 (CN von Cyanomethyl), 94,10 (C-5), 89,17 (C-1′), 82,34 (C-2′), 81,60 (C-4′), 67,27 (C-3′), 59,06 (C-5′), 55,82 (CH₂ von Cyanomethyl), 17,20, 17,08, 16,80 und 16,61 (Isopropyl CH₃ₛ), 13,23, 12,70 und 12,24 p.p.m. (Isopropyl CHₛ).

### Beispiel 6

2′-O-Propylierung wird am besten bewirkt durch 2′-O-Allylierung gefolgt von Reduktion der Allylgruppe. 2′-O-Butylierung wird am besten durchgeführt durch 2′-O-Crotylierung (unter Verwendung von Crotylbromid und dem Verfahren, wie es in Beispiel 4 beschrieben ist), gefolgt von Reduktion der Crotylgruppe.

### Beispiel 7

### 3′,5′-O-(Tetraisopropyldisiloxan-1,3-diyl)-4-O-(2,6-dichlorphenyl)-uridin

14,65 g (60 mmol) getrocknetes Uridin werden in 150 ml wasserfreiem Pyridin gelöst und die Lösung im Eisbad gekühlt. Dazu wird eine Lösung von 21 g (67 mmol) 1,3-Dichlor-1,1,3,3-tetraisopropyldisiloxan in 10 ml Dichlormethan während 15 Minuten unter Rühren und Feuchtigkeitsausschluß zugefügt.Nach beendeter Zugabe wird die Mischung 3 Stunden bei Raumtemperatur weitergerührt; im Dünnschichtchromatogramm (Kieselgel; Fließmittel Chloroform/Ethanol 9:1) beobachtet man danach vollständige Umsetzung zu einem Produkt mit R_{f} 0,58. Die Reaktion wird durch Zugabe von 5 ml Methanol gestoppt und die Mischung im Vakuum eingedampft. Der Rückstand wird in 200 ml Dichlormethan aufgenommen und 2x mit je 200 ml 1 mol/l Natriumbicarbonat-Lösung extrahiert. Die organische Phase wird über Na₂SO₄ getrocknet, filtriert und im Vakuum eingedampft. Der Rückstand wird 2x mit je 25 ml Toluol im Vakuum coevaporiert wonach ein weißer schaumiger Rückstand resultiert. Dieser wird in 200 ml wasserfreiem 1,2-Dichlorethan gelöst und mit 42 ml Triethylamin (300 mmol), sowie 22,5 ml Chlortrimethylsilan (180 mmol) unter Rühren und Feuchtigkeitsausschluß versetzt. Nach 30 Minuten Reaktionszeit zeigt ein Dünnschichtchromatogramm (Kieselgel; Petrolether/Ethylacetat 2:1) vollständige Umsetzung mit einem Fleck vom R_{f} 0,39. Die Reaktionsmischung wird unter kräftigem Rühren in 500 ml 1 mol/l Natriumbicarbonat-Lösung gegossen, die organische Phase abgetrennt und über Na₂SO₄ getrocknet. Nach Filtration wird im Vakuum eingedampft und der Rückstand noch 2x mit je 25 ml trockenem Toluol coevaporiert. Das so erhaltene 2′-O-Trimethylsilyl-Derivat wird in 300 ml wasserfreiem Di-chlormethan gelöst und mit 42 ml Triethylamin (300 mmol), 19,5 g 2-Mesitylensulphonylchlorid (90 mmol) und 1,8 g 4-Dimethylaminopyridin (15 mmol) unter Rühren und Feuchtigkeitsausschluß versetzt. Im DC (Kieselgel; Petrolether/Ethylacetat 2:1) beobachtet man nach 30 minütiger Reaktionszeit vollständige Umsetzung zu einem Produkt mit R_{f} 0,63. Zur Reaktionslösung gibt man 1,35 g 1,4-Diazabicyclo[2.2.2]octan (12 mmol) und 19,6 g 2,6-Dichlorphenol (120 mmol) und rührt 2 Stunden bei Raumtemperatur. Nach dieser Zeit ist die Umsetzung vollständig, wie ein DC in Petrolether/Ethylacetat 2:1 auf Kieselgel zeigt (R_{f} 0,56). Die Reaktionsmischung wird in 500 ml 1 mol/l Na-bicarbonat-Lösung eingerührt die organische Phase abgetrennt, über Na₂SO₄ getrocknet, filtriert und im Vakuum eingedampft. Der 2′-O-Trimetylsilylether wird als öliger, viskoser Rückstand erhalten. Der Sirup wird in 300 ml Dichlormethan gelöst und dazu unter Rühren eine Lösung von 28,5 g p-Toluolsulfonsäure-monohydrat (150 mmol) in 100ml Tetrahydrofuran gegeben. Nach 2,5 Minuten werden 28 ml Triethylamin zur Neutralisation der Säure zugefügt. Danach wird die Reaktionslösung unter starkem Rühren in 500 ml 1 mol/l Na-bicarbonat-Lösung eingegossen. Die organische Phase wird abgetrennt, über Na₂SO₄ getrocknet und das Lösungsmittel im Vakuum abdestilliert. Das DC (Kieselgel; Petrolether/Ethylacetat 1:1) zeigt einen Fleck mit R_{f} 0,59 von 2,6-Dichlorphenyl-2-mesitylensulfonat und einen weiteren mit R_{f} 0,41 des gewünschten Produktes. Das rohe Produkt wird in mehreren Portionen durch präparative Chromatographie an Kieselgel mit Petrolether/Ethylacetat(2:1) als Eluens gereinigt. Nach Eindampfen der Fraktionen erhält man 25,6 g, entsprechend 67,5 % der Theorie an reinem Endprodukt.

### R_{f}-Wert (Kieselgel; Petrolether/Ethylacetat 2:1) 0,23

¹³C NMR-Spektrum (CDCl₃) δ: 169,82 (C-4), 154,70 (C-2), 144,94 (C-6), 144,77 (Phenyl-C-1), 128,92 (Phenyl-C-2 and C-6), 128,71 (Phenyl C-3 und C-5), 127,11 (Phenyl C- 4), 94,05 (C-5), 92,22 (C-1′), 82,01 (C-4′), 74,88 (C-2′), 68,89 (C-3′), 60,35 (C-5′), 17,40 - 16,85 (Isopropyl-CH₃'s), 13,34, 12,91, 12,83 und 12,48 ppm (Isopropyl-CH's).

### Beispiel 8

### 3′,5′-O-(Tetraisopropyldisiloxan-1,3-diyl)-2′-O-allyl-4-O-(2,6-dichlorphenyl)uridin

Tris(dibenzylidenaceton)dipalladium(0) (0,183 g, 0,2 mmol) und 1,4-Bis(diphenylphosphino)butan (0,341 g, 0,8 mmol) werden in trockenem Tetrahydrofuran (40 ml) unter Argonatmosphäre suspendiert. Eine Lösung der nach Beispiel 7 hergestellten Verbindung (12,63 g, 20 mmol) und Allylethylcarbonat (5,2 g, 40 mmol) in trockenem Tetrahydrofuran (60 ml) wird zugegeben und die Mischung für 30 min unter Rückfluß erhitzt. Der vollständige Reaktionsablauf wird durch Dünnschichtchromatographie (Kieselgel, Laufmittel Petrolether/Ethylacetat, 2:1, vol.) überprüft. Das Reaktionsprodukt wird durch eine neue Bande mit einem Rf-Wert von 0,48 angezeigt. Nach Abkühlen wird die Mischung filtriert und das Lösungsmittel im Vakuum entfernt. Das Reaktionsprodukt wird durch präparative Chromatographie an Kieselgel mit 3 % Ethylacetat in Dichlormethan als Laufmittel gereinigt.

Nach Eindampfen der Fraktionen erhält man 11 g (81,9 % der Theorie) Endprodukt.

### R_{f}-Wert (Kieselgel-Dünnschichtchromatographie; Petrolether/Ethylacetat 2:1): 0,51

¹³C NMR-Spektrum (CDCl₃) δ: 169,61 (C-4), 154,49 (C-2), 144,64 (Phenyl C-1), 144,37 (C-6), 134,29 (Allyl CH), 128,75 (Phenyl C-2 und C-6), 128,51 (Phenyl C-3 und C-5), 126,93 (Phenyl C-4), 116,85 (Allyl = CH₂), 93,50 (C- 5), 89,94 (C-1′), 81,64 (C-2′), 80,40 (C-4′), 70,90 (O-CH₂ von Allyl), 67,49 (C-3′), 59,34 (C-5′), 17,24, 17,10, 16,79 und 16,63 (Isopropyl CH₃s), 13,21, 12,83, 12,70 und 12,30 p.p.m. (Isopropyl CHs).

### Beispiel 9

### 2′-O-Allyl-4-O-(2,6-dichlorphenyl)uridin

5,5 g (8,19 mmol), der nach Beispiel 8 hergestellten Verbindung, werden in 20 ml trockenem Tetrahydrofuran gelöst und 1,1 Mol/l Tetrabutylammoniumfluorid in 18 ml Tetrahydrofuran unter Rühren zugegeben. Nach 5 min ist die Reaktion vollständig wie ein Dünnschichtchromatogramm mit Ethanol/Chloroform (5 : 95 vol.) als Laufmittel auf Kieselgel zeigt (R_{f} : 0,22).

Die Reaktion wird mit Pyridin/Methanol/Wasser (50 ml, 3 : 1 : 1 vol.) gestoppt und die Lösung unter Rühren auf die Pyridinform von Dowex 50 Wx4-200 Harz (30 g suspendiert in Pyridin/Methanol/Wasser 50 ml 3: 1 : 1 vol) gegeben. Die Mischung wird 20 min gerührt, das Harz abfiltriert und mit dem o. g. Lösungsmittel (3 x 50 ml) gewaschen. Die vereinigten Filtrate und Waschlösungen werden bis zum Trockenen im Vakuum eingedampft, in Toluol aufgenommen und nochmals eingedampft. Das Rohprodukt wird in 3 Portionen durch präparative Chromatographie auf Kieselgel mit 6 % Ethanol in Chloroform als Elutionsmittel gereinigt. Nach Eindampfen der Fraktionen im Vakuum werden Reste von Ethanol und Pyridin durch Zugabe von Toluol und nochmaliges Eindampfen im Vakuum bei 45°C entfernt. Nach Eindampfen erhält man 2,91 g (82,9 % der Theorie) an reinem Endprodukt.

### R_{f}-Wert (Kieselgel; Ethanol/Chloroform 1 : 4): 0,57

¹³C NMR Spektrum (CDCl₃) δ: 169,74 (C-4); 155,28 (C-2), 146,20 (C-6), 144,42 (phenyl C-1), 133,54 (allyl CH), 128,67 (phenyl C-2 und C-6), 128,57 (phenyl C-3 und C-5), 127,13 (phenyl C-4), 117,98 (allyl = CH₂), 94,41 (C-5), 89,60 (C-1′), 84,54 (C-4′), 81,01 (C-2′), 71,10 (allyl CH₂0), 67,43 (C-3′) and 59,55 p.p.m. (C-5′).

### Beispiel 10

### 2′-O-Allyl-uridin

2,91 g (6,79 mmol) der nach Beispiel 9 hergestellten Verbindung werden in 20 ml trockenem Acetonitril gelöst. Es werden 2,82 g (16,98 mmol) 2-Nitrobenzaldoxim und 1,76 g (15,28 mmol) 1,1,3,3-Tetramethylguanidin in 20 ml trockenem Acetonitril zugegeben und die Mischung bei Raumtemperatur 18 Stunden gerührt. Ein Dünnschichtchromatogramm auf Kieselgel mit Ethanol/Chloroform (1 : 4 vol) als Laufmittel zeigt, daß die Reaktion vollständig abgelaufen ist (R_{f} 0,37). Das Lösungsmittel wird im Vakuum abgedampft und der verbleibende Rest in 100 ml Dichlormethan gelöst und das Produkt mit 100 ml Wasser extrahiert. Die wäßrige Phase wird mit 100 ml Dichlormethan und anschließend mit 100 ml Diethylether gewaschen. Die gelbliche wäßrige Phase wird anschließend mit der Pyridinform von Dowex 50 Wx4-200 Harz (25 g) 5 min gerührt. Das Harz wird abfiltriert und das trübe Filtrat mit 2 x 50 ml Dichlormethan und anschließend 100 ml Ether gewaschen. Die wäßrige Phase wird im Vakuum eingedampft. Wasserspuren werden durch Zugabe von Methanol und Tetrahydrofuran und anschließendes Eindampfen beseitigt. Die gewünschte Verbindung wird aus Methanol kristallisiert, abfiltriert und mit Ether gewaschen und getrocknet. Man erhält 1,83 g (94 % der Theorie) von 2′- O-Allyluridin.

### R_{f}-Wert (Kieselgel; Ethanol/Chloroform 1 : 4 vol): 0,39.

¹³C N.M.R. spektrum (pyridin - d₅ )δ: 164,48 (C-4), 151,72 (C-2), 140,76 (C-6), 135,11 (CH von Allyl), 116,96 (allyl = CH₂), 102,17 (C-5), 88,26 (C-1′), 85,72 (C-4′), 82,57 (C-2′), 71,38 (allyl CH₂O), 69,41 (C-3′) and 60,75 p.p.m. (C-5′).

### Beispiel 11

### 2′-O-Allyl-uridin-5′-monophosphat

1,42 g 2′-O-Allyl-uridin (5,0 mmol) wurden nach der Methode von Yoshikawa et al. (1967) Tetrahedron Lett. 50, 5065 phosphoryliert. Die Ausbeute nach chromatographischer Aufreinigung betrug 980 mg.

### Beispiel 12

### 2′-O-Allyl-uridin-5′-diphosphat und -5′-triphosphat

Zur Herstellung der Di- bzw. Triphosphate wurden jeweils 365 mg (1 mmol) des Monophosphates nach der Methode von Hoard und Ott (1965) J. Am. Chem. Soc. 87, 1785 mit Orthophosphorsäure bzw. Pyrophosphorsäure umgesetzt. Die Ausbeuten betrugen 220 mg an Diphosphat bzw. 140 mg an Triphosphat.

Alle 5′-Phosphate wurden mittels Elementaranalyse, Elektrophorese und ³¹ P-NMR-Spektroskopie charakterisiert.

## Patentansprüche (Patentansprüche für folgende(n) Vertragsstaat(en): AT, BE, CH, LI, DE, DK, FR, GB, GR, IT, LU, NL, SE)

1. Nukleotid-Analogon der allgemeinen Formel I, 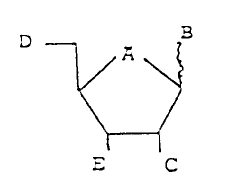 worin
B eine beliebige Nukleobase,
A ein O-Atom oder CH₂;
C gleich -O-R ist und R eine gegebenenfalls modifizierte Alkylgruppe mit insgesamt mindestens 2 C-Atomen, oder eine gegebenenfalls modifizierte Alkenyl- oder Alkinylgruppe mit mindestens 2 C-Atomen bedeutet, wobei die Modifikation in einer Substitution durch einen oder mehrere Halogen-, Cyano-, Carboxy-, Hydroxy-, Nitro- oder/und Mercapto-Reste besteht, und
D und E an sich bekannte, zur Ausbildung von 3′-5′-Internukleotid-Bindungen fähige reaktive Gruppen oder die -PO₄H₂-, -P₂O₇H₃- oder P₃O₁₀H₄-Gruppe bedeuten.

2. Nukleotid nach Anspruch 1,
**dadurch gekennzeichnet,**
daß es als Nukleobase einen Adenin-9-yl-, Cytosin-1-yl-, Guanin-9-yl-, Uracil-1-yl-, Hypoxanthin-9-yl- oder Thymin-1-yl-Rest aufweist.

3. Nukleotid nach Anspruch 1 oder 2,
**dadurch gekennzeichnet,**
daß die Nukleobase ein 2-Aminoadenin-9-yl-Rest ist.

4. Nukleotid nach Anspruch 1 oder 2,
**dadurch gekennzeichnet,**
daß der Alkenylrest ein Alk-2-enylrest ist.

5. Nukleotid nach einem der vorhergehenden Ansprüche,
**dadurch gekennzeichnet,**
daß der Alkenylrest von Allylrest ist.

6. Verfahren zur Herstellung von Poly- und Oligonukleotiden, insbesondere an einer festen Phase,
**dadurch gekennzeichnet,**
daß man mindestens ein Nukleotid nach einem der Ansprüche 1 bis 5 auf an sich bekannte Weise mit anderen Nukleotiden umsetzt.

7. Nukleotid-Polymere, erhältlich durch Umsetzung von reaktiven Mononukleotiden auf an sich bekannte Weise, insbesondere an einer festen Phase,
**dadurch gekennzeichnet,**
daß es mindestens ein Monomeres nach den Ansprüchen 1 bis 5 enthält.

8. Nukleotid-Polymeres nach Anspruch 7 mit der Formel II 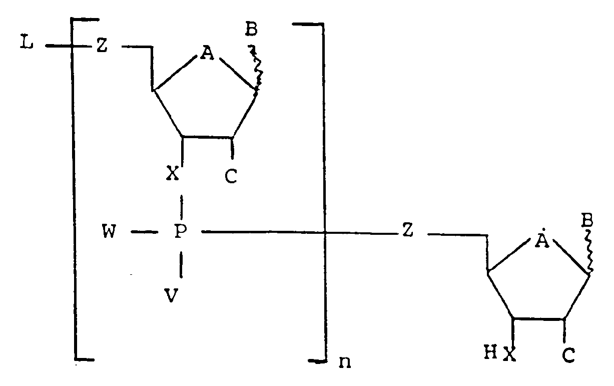 wobei
X oder Z gleich O, S, NH oder CH₂ bedeutet, wobei X und
Z in einer monomeren Einheit gleich oder verschieden sein kann,
V und W ein O, S, Se, NH₂, ein Alkyl- oder ein Alkyloxyrest, OH oder SH bedeutet, wobei V und W in einer Monomereinheit gleich oder verschieden sein können und
L ein H-Atom oder ein Partner eines Bindepaares ist und wobei B, A und C die in Anspruch 1 definierte Bedeutung aufweisen und
n eine beliebige ganze Zahl bedeutet.

9. Nukleotid-Polymeres nach einem der Ansprüche 7 oder 8,
**dadurch gekennzeichnet,**
daß L einen Biotinrest bedeutet.

10. Polynukleotid nach einem der Ansprüche 7 bis 9,
**dadurch gekennzeichnet,**
daß es an seinem 3′-terminalen Ende ein 3′-Deoxyribonukleosid aufweist.

11. Verwendung von Nukleotidpolymeren nach einem der vorhergehenden Ansprüche als Antisense-Proben zur Hemmung der Genexpression und als Arzneimittel.

## Patentansprüche (Patentansprüche für folgende(n) Vertragsstaat(en): ES)

1. Verfahren zur Herstellung eines Nukleotid-Analogons der allgemeinen Formel I, 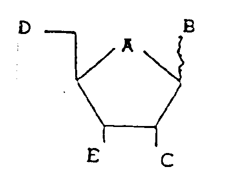 worin
B eine beliebige Nukleobase,
A ein O-Atom oder CH₂;
C gleich -O-R ist und R eine gegebenenfalls modifizierte Alkylgruppe mit insgesamt mindestens 2 C-Atomen, oder eine gegebenenfalls modifizierte Alkenyl- oder Alkinyl-gruppe mit mindestens 2 C-Atomen bedeutet, wobei die Modifikation in einer Substitution durch einen oder mehrere Halogen-, Cyano-, Carboxy-, Hydroxy-, Nitro- oder/und Mercapto-Reste besteht, und
D und E an sich bekannte, zur Ausbildung von 3′-5′-Internukleotid-Bindungen fähige reaktive Gruppen oder die -PO₄H₂-, -P₂O₇H₃ - oder P₃O₁₀H₄-Gruppe bedeuten,
**dadurch gekennzeichnet,**
daß man die 2′-Position eines Nukleotids, in dem A, B, E und D die oben angegebene Bedeutung haben, mit dem Rest C substituiert.

2. Verfahren nach Anspruch 1,
**dadurch gekennzeichnet,**
daß man als Nukleobase einen Adenin-9-yl-, Cytosin-1-yl-, Guanin-9-yl-, Uracil-1-yl, Hypoxanthin-9-yl-, oder Thymin-1-yl-Rest verwendet.

3. Verfahren nach Anspruch 1 oder 2,
**dadurch gekennzeichnet,**
daß die Nukleobase ein 2-Aminoadenin-9-yl-Rest ist.

4. Verfahren nach Anspruch 1 oder 2,
**dadurch gekennzeichnet,**
daß der Alkenylrest ein Alk-2-enylrest ist.

5. Verfahren nach einem der vorhergehenden Ansprüche,
**dadurch gekennzeichnet,**
daß der Alkenylrest ein Allylrest ist.

6. Verfahren zur Herstellung von Poly- und Oligonukleotiden, insbesondere an einer festen Phase,
**dadurch gekennzeichnet,**
daß man mindestens ein nach einem der Ansprüche 1 bis 5 erhaltenes Nukleotid auf an sich bekannte Weise mit anderen Nukleotiden umsetzt.

7. Verfahren nach Anspruch 6,
**dadurch gekennzeichnet,**
daß man ein Polymeres mit der Formel II 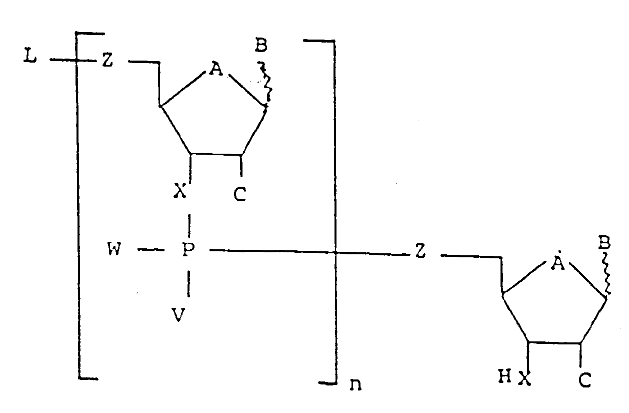 wobei
X oder Z gleich O, S, NH oder CH₂ bedeutet, wobei X und
Z in einer monomeren Einheit gleich oder verschieden sein kann,
V und W ein O, S, Se, NH₂, ein Alkyl- oder ein Alkyloxyrest, OH oder SH bedeutet, wobei V und W in einer Monomereinheit gleich oder verschieden sein können und
L ein H-Atom oder ein Partner eines Bindepaares ist und wobei B, A und C die in Anspruch 1 definierte Bedeutung aufweisen und
n eine beliebige ganze Zahl bedeutet, herstellt.

8. Verfahren nach einem der Ansprüche 6 oder 7,
**dadurch gekennzeichnet,**
daß L einen Biotinrest bedeutet.

9. Verfahren nach einem der Ansprüche 6 bis 8,
**dadurch gekennzeichnet,**
daß das Polynukleotid an seinem 3′-terminalen Ende ein 3′-Deoxyribonukleosid aufweist.

10. Verwendung von Nukleotidpolymeren nach einem der vorhergehenden Ansprüche als Antisense-Proben zur Hemmung der Genexpression und als Arzneimittel.

## Claims (Claims for the following Contracting State(s): AT, BE, CH, DE, DK, FR, GB, GR, IT, LI, LU, NL, SE)

1. Nucleotide analogue having the general formula I, 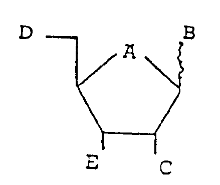 in which
B is an arbitrary nucleobase,
A is an O atom or CH₂;
C equals -O-R and R denotes an alkyl group with a total of at least 2 C atoms which may be modified if desired, or it denotes an alkenyl group or an alkinyl group with at least 2 C atoms which may be modified if desired, in which the modification consists of a substitution by one or several halogen, cyano, carboxy, hydroxy, nitro or/and mercapto residues, and
D and E denote known reactive groups capable of forming 3′-5′ internucleotide bonds or denote a -PO₄H₂, -P₂O₇H₃ or -P₃O₁₀H₄ group.

2. Nucleotide as claimed in claim 1,
**wherein**
it has an adenin-9-yl, cytosin-1-yl, guanin-9-yl, uracil-1-yl, hypoxanthin-9-yl or thymin-1-yl residue as the nucleobase.

3. Nucleotide as claimed in claim 1 or 2,
**wherein**
the nucleobase is a 2-aminoadenin-9-yl residue.

4. Nucleotide as claimed in claim 1 or 2,
**wherein**
the alkenyl residue is an alk-2-enyl residue.

5. Nucleotide as claimed in one of the previous claims,
**wherein**
the alkenyl residue is an allyl residue.

6. Process for the production of polynucleotides and oligonucleotides, in particular on a solid phase,
**wherein**
at least one nucleotide as claimed in one of the claims 1 to 5 is reacted with other nucleotides in a known manner.

7. Nucleotide polymers obtainable by reacting reactive mononucleotides in a known manner, in particular on a solid phase,
**wherein**
it contains at least one of the monomers as claimed in claims 1 to 5.

8. Nucleotide polymer as claimed in claim 7 having the formula II 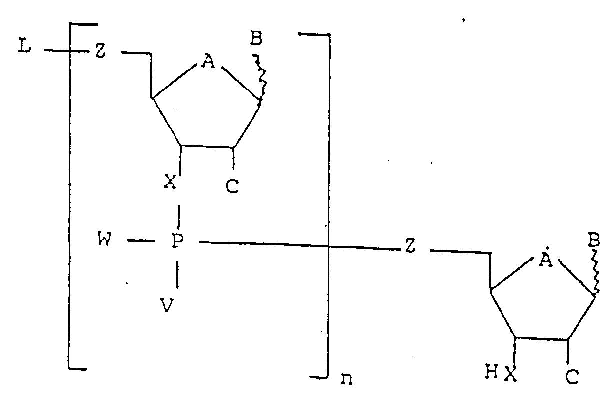 in which
X or Z equal O, S, NH or CH₂ whereby X and Z can be the same or different in a monomer unit,
V and W denote O, S, Se, NH₂, an alkyl or alkyloxy residue, OH or SH whereby V and W can be the same or different in a monomer unit and
L is an H atom or a partner of a binding pair and in which B, A and C have the meaning defined in claim 1 and
n denotes an arbitrary integer.

9. Nucleotide polymer as claimed in one of the claims 7 or 8,
**wherein**
L denotes a biotin residue.

10. Polynucleotide as claimed in one of the claims 7 to 9,
**wherein**
it has a 3′-deoxyribonucleoside at its 3′-terminal end.

11. Use of nucleotide polymers as claimed in one of the previous claims as antisense probes for inhibiting gene expression and as pharmaceutical agents.

## Claims (Claims for the following Contracting State(s): ES)

1. Process for the production of a nucleotide analogue having the general formula I, 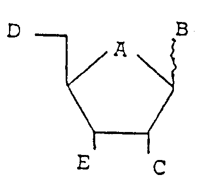 in which
B is an arbitrary nucleobase,
A is an O atom or CH₂;
C equals -O-R and R denotes an alkyl group with a total of at least 2 C atoms which may be modified if desired, or it denotes an alkenyl group or an alkinyl group with at least 2 C atoms which may be modified if desired, in which the modification consists of a substitution by one or several halogen, cyano, carboxy, hydroxy, nitro or/and mercapto residues, and
D and E denote known reactive groups capable of forming 3′-5′ internucleotide bonds or denote a -PO₄H₂, -P₂O₇H₃ or -P₃O₁₀H₄ group,
**wherein**
the 2′ position of a nucleotide, in which A, B, E and D have the aforementioned meaning, is substituted with the residue C.

2. Process as claimed in claim 1,
**wherein**
an adenin-9-yl, cytosin-1-yl, guanin-9-yl, uracil-1-yl, hypoxanthin-9-yl or thymin-1-yl residue is used as the nucleobase.

3. Process as claimed in claim 1 or 2,
**wherein**
the nucleobase is a 2-aminoadenin-9-yl residue.

4. Process as claimed in claim 1 or 2,
**wherein**
the alkenyl residue is an alk-2-enyl residue.

5. Process as claimed in one of the previous claims,
**wherein**
the alkenyl residue is an allyl residue.

6. Process for the production of polynucleotides and oligonucleotides, in particular on a solid phase,
**wherein**
at least one nucleotide obtained as claimed in one of the claims 1 to 5 is reacted with other nucleotides in a known manner.

7. Process as claimed in claim 6,
**wherein**
a polymer having the formula II 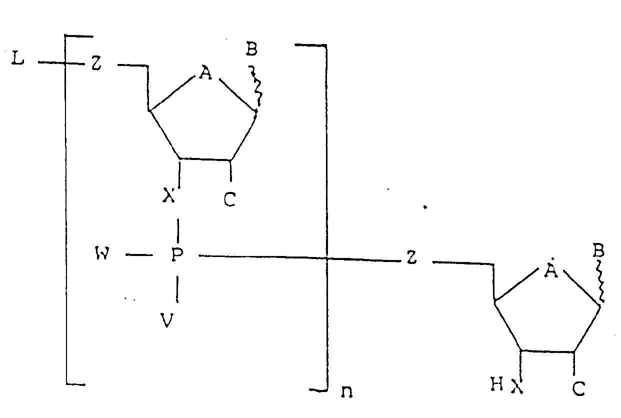 in which
X or Z equal O, S, NH or CH₂ whereby X and Z can be the same or different in a monomer unit,
V and W denote O, S, Se, NH₂, an alkyl or alkyloxy residue, OH or SH whereby V and w can be the same or different in a monomer unit and
L is an H atom or a partner of a binding pair and in which B, A and C have the meaning defined in claim 1 and
n denotes an arbitrary integer, is produced.

8. Process as claimed in one of the claims 6 or 7,
**wherein**
L denotes a biotin residue.

9. Process as claimed in one of the claims 6 to 8,
**wherein**
the polynucleotide has a 3′-deoxyribonucleoside at its 3′-terminal end.

10. Use of nucleotide polymers as claimed in one of the previous claims as antisense probes for inhibiting gene expression and as pharmaceutical agents.

## Revendications (Revendications pour l'(les) Etat(s) contractant(s) suivant(s): AT, BE, CH, DE, DK, FR, GB, GR, IT, LI, LU, NL, SE)

1. Analogon-nucléotide de formule générale I, 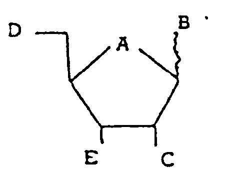 dans laquelle
B est une quelconque nucléobase,
A est un atome O ou CH₂ ;
C est identique à -O-R et signifie un groupe alkyle éventuellement modifié avec au total au moins 2 atomes C ou un groupe alcényle ou alkynyle éventuellement modifié avec 2 atomes C, ce en quoi la modification consiste en une substitution par un ou plusieurs radicaux halogène, cyano, carboxy, hydroxy, nitro ou/et mercapto, et
D et E connus per se signifient des groupes réactifs ou le groupe -PO₄H₂-, -P₂O₇H₃- ou P₃O₁₀H₄- aptes à la formation de liaisons 3′-5′-internucléotides.

2. Nucléotide selon la revendication 1, caractérisé en ce qu'il présente en tant que nucléobase un radical adénin-9-yl-, cytosin-1-yl-, guanin-9-yl-, uracil-1-yl-, hypoxanthin-9-yl- ou thymin-1-yle.

3. Nucléotide selon la revendication 1 ou 2, caractérisé en ce que la nucléobase est un radical 2-aminoadénin-9-yle.

4. Nucléotide selon la revendication 1 ou 2, caractérisé en ce que le radical alcényle est un radical alc-2-ényle.

5. Nucléotide selon l'une des revendications précédentes, caractérisé en ce que le radical alcényle est un radical allyle.

6. Procédé pour la préparation de poly- et oligonucléotides, notamment sur une phase solide, caractérisé en ce que l'on fait réagir au moins un nucléotide selon l'une des revendications 1 à 5 d'une manière connue per se avec d'autres nucléotides.

7. Nucléotide-polymère, pouvant être obtenu par la réaction de mononucléotides réactifs d'une manière connue per se, notamment en phase solide, caractérisé en ce qu'il contient au moins un monomère selon les revendications 1 à 5.

8. Nucléotide-polymère selon la revendication 7, avec la formule II 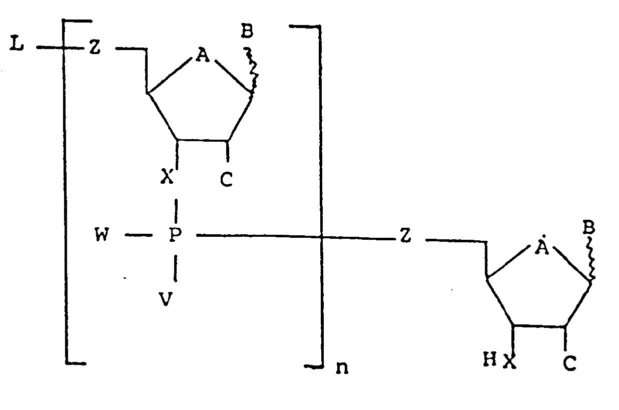 dans laquelle
X ou Z sont identiques, signifient O, S, NH ou CH₂, ce en quoi X et Z peuvent être identiques ou différents dans une unité monomère,
V et W signifient un O, S, Se, NH₂, un radical alkyle ou un radical alkyloxy, signifient OH ou SH, ce en quoi V et W peuvent être identiques ou différents dans une unité monomère et
L est un atome H ou un partenaire d'un couple de liaison et ce en quoi B, A et C ont la signification définie dans la revendication 1, et
n signifie un quelconque nombre entier.

9. Polymères-nucléotides selon l'une des revendications 7 ou 8, caractérisé en ce que L signifie un radical biotine.

10. Polynucléotide selon l'une des revendications 7 à 9, caractérisé en ce qu'il présente sur son extrémité 3′ terminale un 3′-désoxyribonucléoside.

11. Utilisation de polymères nucléotides selon l'une des revendications précédentes, en tant que sondes anti-sens pour l'inhibition de l'expression du gène et comme médicaments.

## Revendications (Revendications pour l'(les) Etat(s) contractant(s) suivant(s): ES)

1. Procédé pour la préparation d'un analogonnucléotide de la formule générale I, 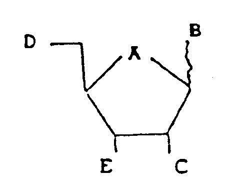 dans laquelle
B est une quelconque nucléobase,
A est un atome O ou CH₂ ;
C est identique à -O-R et R signifie un groupe alkyle éventuellement modifié avec au total au moins 2 atomes C ou un groupe alcényle ou alkynyle éventuellement modifié avec 2 atomes C, ce en quoi la modification consiste en une substitution par un ou plusieurs radicaux halogène, cyano, carboxy, hydroxy, nitro ou/et mercapto, et
D et E connus per se signifient des groupes réactifs ou le groupe -PO₄H₂-, -P₂O₇H₄- ou P₃O₁₀H₄- aptes à la formation de liaisons 3′-5′-internucléotides,
caractérisé en ce que l'on substitue la position 2′ d'un nucléotide, dans lequel A, B, E et D ont la signification indiquée ci-dessus, par le radical C.

2. Procédé selon la revendication 1, caractérisé en ce que l'on utilise comme nucléobase un radical adénin-9-yl, cytosin-1-yl-, guanin-9-yl-, uracil-1-yl-, hypoxanthin-9-yl-ou thymin-1-yle.

3. Procédé selon la revendication 1 ou 2, caractérisé en ce que la nucléobase est un radical 2-aminoadénin-9-yle.

4. Procédé selon la revendication 1 ou 2, caractérisé en ce que le radical alcényle est un radical alc-2-ényle.

5. Procédé selon l'une des revendications précédentes, caractérisé en ce que le radical alcényle est un radical allyle.

6. Procédé pour la préparation de poly- et d'oligo-nucléotides, notamment dans une phase solide, caractérisé en ce que l'on fait réagir au moins un nucléotide obtenu selon l'une des revendications 1 à 5 d'une manière connue per se avec d'autres nucléotides.

7. Procédé selon la revendication 6, caractérisé en ce que l'on produit un polymère de formule II 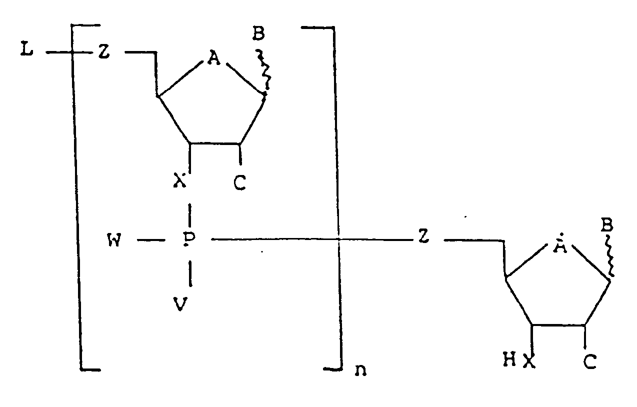 dans laquelle
X ou Z sont identiques, signifient O, S, NH ou CH₂, ce en quoi X et Z peuvent être identiques ou différents dans une unité monomère,
V et W signifient un O, S, Se, NH₂, un radical alkyle ou un radical alkyloxy, signifient OH ou SH, ce en quoi V et W peuvent être identiques ou différents dans une unité monomère et
L est un atome H ou un partenaire d'un couple de liaison et ce en quoi B, A et C ont la signification définie dans la revendication 1, et
n signifie un quelconque nombre entier.

8. Procédé selon l'une des revendications 6 ou 7, caractérisé en ce que L signifie un radical biotine.

9. Procédé selon l'une des revendications 6 à 8, caractérisé en ce que le polynucléotide présente sur son extrémité 3' terminale un 3′-désoxyribonucléoside.

10. Utilisation de polymères nucléotides selon l'une des revendications précédentes, en tant qu'échantillons anti-sens pour l'inhibition de l'expression du gène et comme médicaments.
